# EUROPEAN PATENT APPLICATION

(11) **EP 4 442 782 A1**
(43) Date of publication of application: **09.10.2024**
(21) Application number: 24163018.5
(22) Date of filing: 12.03.2024
(51) Int. Cl.: C09J 4/00

(54) **METHOD FOR ACCELERATING CURING OF MONOMER AND INSTANT ADHESIVE KIT**

(30) Priority: 24.03.2023 JP 2023047434; 05.02.2024 JP 2024015416
(71) Applicant: ThreeBond Co., Ltd., Tokyo 192-0398 (JP)
(72) Inventor: KAWAMOTO, Ippei, Tokyo, 192-0398 (JP); MOTOKI, Shigekazu, Tokyo, 192-0398 (JP)
(74) Representative: Crow, Martin

(57) **Abstract**

The present disclosure provides a curing accelerator that ensures curing acceleration performance while inhibiting whitening and is mildly irritating. The present disclosure relates to a method for accelerating curing of a monomer, the method including curing a monomer using a curing accelerator that contains a component (A) and a component (B) below and does not contain a solvent having a boiling point higher than 120°C:
the component (A): a solvent having 2 or more carbon atoms in one molecule and having a boiling point of 25 to 120°C; and
the component (B): water.

## Description

### BACKGROUND

### 1. Technical Field

The present invention relates to a curing accelerator used in combination with, for example, a cyanoacrylate composition.

### 2. Description of Related Arts

In recent years, adhesives friendly to human bodies and environment are desired. Cyanoacrylate compositions are used in surgery, and in recent years, it is known that there are many people who experience application of eyelash extensions to adhere artificial fibers such as artificial eyelashes to human eyelashes. Since the eyelash extensions are applied near the eyes, an adhesive that is mildly irritating as in JP 2017-075110 A is desired. Unfortunately, as for the characteristics of the cyanoacrylate composition, as it is known that the component volatilizes and whitens, a phenomenon in which the component volatilizes and cures around an application region is known. Even for the cyanoacrylate composition that is originally highly reactive, it is desired to shorten the curing time due to a demand for shortening the time required for application. However, when the curing time is shortened, there is a problem that the storage stability of the cyanoacrylate composition is extremely lowered. Meanwhile, there is a trade-off relationship that when a highly irritating curing accelerator is reduced, curability is deteriorated.

As a solution to these problems, it is known that a curing accelerator component for the cyanoacrylate composition is compounded as another composition functioning as a curing accelerator, and is used in combination with the cyanoacrylate composition. However, even with such a technique, problems of whitening and irritation remain, and in a case where an engineering plastic or the like is used as an adherend, there is another problem that erosion occurs to cause cracks in the worst case.

### SUMMARY

Conventionally, a curing accelerator for a cyanoacrylate composition contains a highly irritating component that affects the human body, and it has been difficult to ensure curing acceleration performance while inhibiting whitening and additionally achieve mildly irritating performance.

Therefore, an object of the present invention is to provide a curing accelerator that ensures curing acceleration performance while inhibiting whitening and is mildly irritating.

As a result of intensive studies to achieve the above object, the present inventors have found a method related to a curing accelerator that does not affect the human body, and have completed the present invention.

The gist of the present invention is described below.
[1] Use of a composition that contains a component (A) and a component (B) below and does not contain a solvent having a boiling point higher than 120°C in accelerating curing of a monomer:
   the component (A): a solvent having 2 or more carbon atoms in one molecule and having a boiling point of 25 to 120°C; and
   the component (B): water;
[2] The use according to [1], wherein the composition consists only of the component (A) and the component (B);
[3] The use according to [1] or [2], wherein the component (A) contains one or more selected from the group consisting of an alcohol, a ketone compound, and an ester compound.;
[4] The use according to [3], wherein the component (A) contains ethanol, n-propanol, n-butanol, or acetone;
[5] The use according to any one of [1] to [3], wherein a mass ratio of the component (A) to the component (B) in the composition is 10 : 90 to 50 : 50;
[6] The use according to any one of [1] to [5], wherein a pH of the component (B) is higher than 7;
[7] The use according to any one of [1] to [6], wherein the monomer includes a cyanoacrylate compound;
[8] The use according to any one of [1] to [7], wherein the curing is performed by applying the monomer and the composition are applied to the human body;
[9] The use according to [8], wherein the curing is performed by applying the monomer and the composition are applied to the human eyelashes;
[10] An instant adhesive kit comprising:
   a composition that contains a component (A) and a component (B) below and does not contain a solvent having a boiling point higher than 120°C; and
   a composition containing a monomer:
      the component (A): a solvent having 2 or more carbon atoms in one molecule and having a boiling point of 25 to 120°C; and
      the component (B): water;
[11] The instant adhesive kit according to [10], wherein the composition consists only of the component (A) and the component (B);
[12] The instant adhesive kit according to [10] or [11], wherein the component (A) contains one or more selected from the group consisting of an alcohol, a ketone compound, and an ester compoun;
[13] The instant adhesive kit according to any one of [10] to [12], wherein a mass ratio of the component (A) to the component (B) in the composition is 10 : 90 to 50 : 50;
[14] The instant adhesive kit according to any one of [10] to [13], wherein a pH of the component (B) is higher than 7;
[15] The instant adhesive kit according to any one of [10] to [14], wherein the monomer includes a cyanoacrylate compound.

According to the present invention, there is provided a curing accelerator that ensures curing acceleration performance while inhibiting whitening and is mildly irritating.

### DETAILED DESCRIPTION

In the present invention, a numerical range may be expressed by "to", and this expression indicates a numerical range including an upper limit and a lower limit.

Details of the present invention are described below. An aspect of the present invention is a curing accelerator that contains a component (A) and a component (B) below and does not contain a solvent having a boiling point higher than 120°C:
the component (A): a solvent having 2 or more carbon atoms in one molecule and having a boiling point of 25 to 120°C; and
the component (B): water.

The component (A) used in the curing accelerator according to the present invention is a solvent having 2 or more carbon atoms in one molecule and having a boiling point of 25 to 120°C. Since a moderately wet state is maintained by use of the component (A), it is considered that when an object to be cured (for example, a cyanoacrylate composition) comes into contact with the curing accelerator, the component (B) to be described later penetrates into the object to be cured to promote curing. The upper limit of the number of carbon atoms in one molecule of the component (A) as the solvent is not particularly limited, but is usually 10 or less, preferably 6 or less, more preferably 5 or less, and still more preferably 4 or less. Therefore, the number of carbon atoms in one molecule of the component (A) as the solvent is preferably 2 to 6, more preferably 2 to 5, and still more preferably 2 to 4. In addition, a solvent having one carbon atom in one molecule (for example, methanol) is not essential. Rather, from the viewpoint of inhibiting whitening, the curing accelerator according to the present invention preferably does not contain a solvent having one carbon atom in one molecule, such as methanol.

Examples of the component (A) include solvents such as alcohols, ketone compounds, and ester compounds, but are not limited thereto. In addition, as long as the number of carbon atoms in one molecule is 2 or more and the boiling point is 25 to 120°C, the component (A) can be used alone or in combination of two or more kinds.

Specific examples of the alcohol include ethanol, n-propanol, and n-butanol, but are not limited thereto. Specific examples of the ketone compound include acetone and methyl ethyl ketone, but are not limited thereto. Specific examples of the ester compound include methyl acetate and ethyl acetate, but are not limited thereto. Among them, the component (A) preferably contains ethanol, n-propanol, n-butanol, or acetone from the viewpoint of particularly exhibiting the action and effect of the present invention.

Here, the curing accelerator according to the present invention is also characterized in that it does not contain a solvent having a boiling point higher than 120°C. This is because the incorporation of such a solvent may erode an adherend on which a cured product is formed and damage the adherend.

The component (B) used in the curing accelerator according to the present invention is water. In particular, the pH of the component (B) is preferably 6 or more, more preferably higher than 7, and still more preferably 8 or more. On the other hand, the upper limit of the pH of the component (B) is not particularly limited, but the pH is preferably 12 or less. When the pH is on the alkaline side, there is an advantage that curing is further accelerated.

Specific examples of the component (B) include ion-exchanged water and alkaline electrolyzed water, but are not limited thereto. The component (B) is most preferably alkaline electrolyzed water from the viewpoint that the pH is higher than 7 to improve the curing acceleration performance. In general, the alkaline electrolyzed water is produced by electrolyzation of an aqueous sodium chloride solution or an aqueous calcium carbonate solution in a two-chamber or three-chamber electrolytic bath and is produced as water generated on the cathode side, but the production method is not limited to such a method.

In order to exhibit curing acceleration performance while inhibiting whitening, the mass ratio of the component (A) to the component (B) is preferably 10 : 90 to 90 : 10, more preferably 15 : 85 to 50 : 50, still more preferably 20 : 80 to 40 : 60, and most preferably 25 : 75 to 30 : 70.

In addition to the component (A) and the component (B), other accelerator components that accelerate curing may be added as long as the characteristics of the present invention are not impaired, but do not have to be added. Most preferred is a curing accelerator consisting only of the component (A) and the component (B) .

Examples of the other accelerator components include silane coupling agents, mercaptan compounds, carboxylic acids, amine compounds, crown ethers, polyethylene glycol derivatives, and calixarene derivatives, but are not limited thereto.

Specific examples of the silane coupling agent include aminomethyltrimethoxysilane, 3-aminopropyltrimethoxysilane, 2-aminoethylaminomethyl trimethoxysilane, 3-aminopropyldimethylethoxysilane, 3-aminopropyldiethoxymethylsilane, 3-(2-aminoethylaminopropyl)dimethoxymethylsilane, 3-(2-aminoethylaminopropyl)trimethoxysilane, 2-(2-aminoethylthioethyl)diethoxymethylsilane, dimethoxymethyl-2-pyridinoethylsilane, 3-morpholinopropyltrimethoxysilane, dimethoxymethyl-3-piperazinopropylsilane, 3-piperazinopropyltrimethoxysilane, N-(3-triethoxysilylpropyl)urea, 3-aminopropyltriethoxysilane, 3-dimethylaminopropyldiethoxymethylsilane, 2-(2-aminoethylthioethyl)triethoxysilane, 3-[2-(2-aminoethylaminoethylamino)propyl]trimethoxysilane, 3-phenylaminopropyltrimethoxysilane, 2-aminoethylaminomethyl benzyloxydimethylsilane, N-(3-diethoxymethylsilylpropyl)succinimide, 3-cyclohexylaminopropyltrimethoxysilane, 3-cyclohexylaminopropyltriethoxysilane, benzylidene-3-ethoxydimethylsilylpropylamine, dimethoxyphenyl-2-piperidinoethoxysilane, N-(3-triethoxysilylpropyl)-p-nitrobenzamide, and 3-(vinylbenzylaminopropyl)triethoxysilane, but are not limited thereto. One or more of these silane coupling agents may be contained in the curing accelerator, but do not have to be contained, and are preferably not contained.

Specific examples of the mercaptan compound include mercaptoacetic acid, mercaptosuccinic acid, mercaptomalic acid, 3-mercaptopropionic acid, thioglycerol, polyethylene glycol dimercaptoacetate, 2,2'-dimethylmercaptodiethyl ether, thioglycerol, polyethylene glycol di-3-mercaptopropionate, polyethylene glycol dimercaptoacetate, dodecylthioglycolate, methylthioglycolate, 2-mercaptobenzothiazole, diisopropylbenzothiazyl-2-sulfonamide, 2-thio-hydantoin, 2-mercapto-6-nitrobenzothiazole, thiophenol, 2-benzimidazole thiol, 3,3-thiopropionic acid, sodium thioglycolate, polyethylene dimercaptoacetate, isodecylthioglycolate, mercaptosilane, dodecanethiol, mercaptomethyl trimethoxysilane, mercaptomethyl trimethylsilane, dimethoxy-3-mercaptopropylmethylsilane, 3-mercaptopropyltrimethoxysilane, and 1,3-bis(3-mercaptopropyl)tetramethyldisiloxane, but are not limited thereto. One or more of these mercaptan compounds may be contained in the curing accelerator, but do not have to be contained, and are preferably not contained.

Specific examples of the carboxylic acid include oxalic acid, malonic acid, succinic acid, tartaric acid, glutaric acid, adipic acid, maleic acid, fumaric acid, phthalic acid, isophthalic acid, terephthalic acid, formic acid, acetic acid, propionic acid, butyric acid, valeric acid, caproic acid, caprylic acid, lauric acid, myristic acid, palmitic acid, stearic acid, oleic acid, linoleic acid, linolenic acid, cyclohexanecarboxylic acid, phenylacetic acid, benzoic acid, toluic acid, chlorobenzoic acid, bromobenzoic acid, nitrobenzoic acid, phthalic acid, isophthalic acid, salicylic acid, and anthranilic acid, but are not limited thereto. One or more of these carboxylic acids may be contained in the curing accelerator, but do not have to be contained, and are preferably not contained.

Specific examples of the amine compound include 3-aminopropyltriethoxysilane, 3-dimethylaminopropyldiethoxymethylsilane, 2-(2-aminoethylthioethyl)triethoxysilane, 3-[2-(2-aminoethylaminoethylamino)propyl]trimethoxysilane, 3-phenylaminopropyltrimethoxysilane, 2-aminoethylaminomethyl benzyloxydimethylsilane, N-(3-diethoxymethylsilylpropyl)succinimide, 3-cyclohexylaminopropyltrimethoxysilane, 3-cyclohexylaminopropyltriethoxysilane, benzylidene-3-ethoxydimethylsilylpropylamine, alanine, arginine, asparagine, aspartic acid, cysteine, cystine, glutamic acid, glycine, histidine, hydroxylysine, hydroxyproline, isoleucine, leucine, lysine, methionine, phenylalanine, proline, serine, threonine, thyroxin, tryptophan, tyrosine, and valine, but are not limited thereto. One or more of these amine compounds may be contained in the curing accelerator, but do not have to be contained, and are preferably not contained.

The addition amount of the other accelerator components is, for example, 0.001 to 10 parts by mass, and may be 0.001 to 1.0 part by mass with respect to 100 parts by mass in total of the component (A) and the component (B). When the addition amount of the other accelerator components is 10 parts by mass or less, the odor is low.

The curing accelerator according to the present invention does not cure itself. The curing accelerator according to the present invention merely accelerates the curing of an object to be cured, such as a cyanoacrylate composition. As described above, the curing accelerator according to the present invention is preferably used for curing a cyanoacrylate composition containing a cyanoacrylate compound as a monomer (polymerizable monomer). Hereinafter, the cyanoacrylate composition is described, but other objects to be cured can also be used.

The cyanoacrylate compound that is the main component (polymerizable monomer) of the cyanoacrylate composition is represented by the following general formula 1. Here, R in the formula is an organic group, and specific examples thereof include an alkyl group, an alkenyl group, a cyclohexyl group, an aryl group, and an alkoxyalkyl group, and more specific examples thereof include a methyl group, an ethyl group, a n-propyl group, a n-butyl group, an isobutyl group, a n-pentyl group, an allyl group, a cyclohexyl group, a benzyl group, and a methoxypropyl group, but are not limited thereto. The cyanoacrylate compound may be used alone or as a mixture of two or more kinds. From the viewpoint of inhibiting whitening, R is preferably an alkoxyalkyl group.

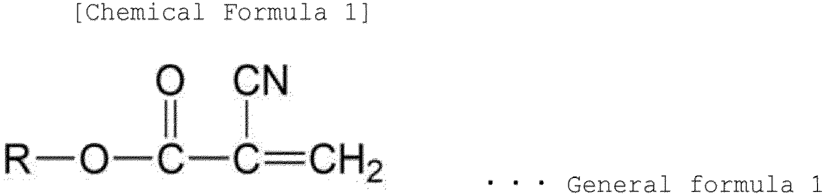

In the cyanoacrylate composition, additives such as a coupling agent, an inorganic filler and an organic filler, a colorant such as a pigment or a dye, an antioxidant, a polymerization inhibitor, an antifoaming agent, a leveling agent, and a rheology control agent may be blended in an appropriate amount as long as the characteristics are not impaired, but the additives do not have to be blended. By the addition of these additives, a composition excellent in resin strength, workability, storage stability, and the like, or a cured product thereof is obtained.

The curing accelerator according to the present invention is a curing accelerator in consideration of reducing adverse effects on the human body and the environment, and is preferably used for uses in which the curing accelerator is applied to the human body. In particular, eyelash extensions applied to human eyelashes are preferably mentioned as a use in which the curing accelerator is applied to the human body. However, the curing accelerator can also be suitably used for other uses such as surgery performed using an adhesive containing a cyanoacrylate composition.

The eyelash extension using the curing accelerator according to the present invention and the cyanoacrylate composition is generally applied in steps 1 to 5 below.
step 1: In a state where the eyes are closed, the lower eyelashes are fixed using an adhesive tape so that almost all of the lower eyelashes are hidden.
step 2: Any dirt attached to the upper eyelashes is removed with a detergent or a solvent applied to a cotton swab or the like.
step 3: The curing accelerator is lightly rubbed and applied to the upper eyelashes using a cotton swab or the like.
step 4: Artificial eyelashes are pinched with tweezers, which are forceps dedicated to eyelash extensions, and the artificial eyelashes are adhered to the upper eyelashes picked up 1 to 1.5 mm from the skin using the cyanoacrylate composition.
step 5: Air blowing or the like is performed to sufficiently cure the cyanoacrylate composition.

Other embodiments of the present invention include the following.
(1) A method for accelerating curing of a monomer, the method including:
   curing a monomer using a curing accelerator that contains a component (A) and a component (B) below and does not contain a solvent having a boiling point higher than 120°C:
   the component (A): a solvent having 2 or more carbon atoms in one molecule and having a boiling point of 25 to 120°C; and
   the component (B): water.
(2) Use of a composition that contains a component (A) and a component (B) below and does not contain a solvent having a boiling point higher than 120°C in accelerating curing of a monomer:
   the component (A): a solvent having 2 or more carbon atoms in one molecule and having a boiling point of 25 to 120°C; and
   the component (B): water.
(3) An instant adhesive kit including:
   a composition (that is, a curing accelerator) that contains a component (A) and a component (B) below and does not contain a solvent having a boiling point higher than 120°C; and
   a composition containing a monomer:
      the component (A): a solvent having 2 or more carbon atoms in one molecule and having a boiling point of 25 to 120°C; and
      the component (B): water.

### Examples

Hereinafter, the present invention is described in more detail with reference to examples, but the present invention is not limited only to these examples.

### [Examples 1 to 6 and Comparative Examples 1 to 9]

In order to prepare Examples 1 to 6 and Comparative Examples 1 to 9, the following components were provided.

Component (A): a solvent having 2 or more carbon atoms in one molecule and having a boiling point of 25 to 120°C
·n-Butanol (boiling point: 118°C) (reagent)
·n-Propanol (boiling point: 97°C) (reagent)
·Acetone (boiling point: 56°C) (reagent)
·Ethanol (boiling point: 78°C) (reagent)

### Component (A) : a solvent other than the component (A)

·Methanol (boiling point: 64°C) (reagent)
·4-Hydroxy-4-methyl-2-pentanone (boiling point: 168°C) (reagent)
·Hydroxyacetone (boiling point: 145°C) (reagent)

### Component (B): water

·Water treated with potassium carbonate (alkaline electrolyzed water) (pH: 12) (ThreeBond 6658 manufactured by ThreeBond Co., Ltd.)
·Purified water (pH: 7) (purified water manufactured by KYOEI PHARMACEUTICAL CO., LTD.)

The component (A) (and/or the component (A')) and the component (B) were weighed in a beaker and stirred for 5 minutes. The detailed preparation amounts are in accordance with Table 1, and all numerical values are expressed in parts by mass.

**[Table 1]**

| Component | Raw material | Example 1 | Example 2 | Example 3 | Example 4 | Example 5 | Example 6 | Comparative Example 1 | Comparative Example 2 |
|---|---|---|---|---|---|---|---|---|---|
| Component (A) | Butanol | | | | | | | 4 | |
| | Propanol | 4 | | | 4 | | | | 4 |
| | Acetone | | 4 | | | 4 | | | |
| | Ethanol | | | 4 | | | 4 | | |
| Component (A') | Methanol | | | | | | | | |
| | 4-Hydroxy-4-methyl-2-pentanone | | | | | | | 6 | 6 |
| | Hydroxyacetone | | | | | | | | |
| Component (B) | Alkaline electrolyzed water | 10 | 10 | 10 | | | | 10 | 10 |
| | Purified water | | | | 10 | 10 | 10 | | |
| Total | | 14.0 | 14.0 | 14.0 | 14.0 | 14.0 | 14.0 | 20.0 | 20.0 |

| Component | Raw material | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|
| Component (A) | Butanol | | | | 4 | 4 | 4 | 4 |
| | Propanol | | | | | | | |
| | Acetone | 4 | | | | | | |
| | Ethanol | | | | | | | |
| Component (A') | Methanol | | 4 | 4 | 12 | | | |
| | 4-Hydroxy-4-methyl-2-pentanone | 6 | 6 | | | 6 | 6 | 6 |
| | Hydroxyacetone | | | 7.6 | | | | |
| Component (B) | Alkaline electrolyzed water | 10 | 10 | 12.5 | 10 | | 1 | |
| | Purified water | | | | | 10 | | 1 |
| Total | | 20.0 | 20.0 | 24.1 | 26.0 | 20.0 | 11.0 | 11.0 |

Evaluation of the appearance 1 of the instant adhesive, the appearance 2 of the instant adhesive, and the appearance of the adherend, and measurement of the setting time were performed using Examples 1 to 6 and Comparative Examples 1 to 9 as curing accelerators in combination with a cyanoacrylate composition by the following methods. In addition, the same test was performed using only the cyanoacrylate composition as Reference Example 1. The results are summarized in Table 2.

### [Appearance of cured product of cyanoacrylate composition]

On a steel sheet 2.0 mm thick × 25 mm wide × 100 mm long, 7 mg of ThreeBond 7737 manufactured by ThreeBond Co., Ltd. is dropped as a cyanoacrylate composition, and immediately, about 10 mg of a curing accelerator is dropped inside a range in which the cyanoacrylate composition has been dropped. The appearance in an atmosphere of 25°C and 55% RH is judged according to the following evaluation criteria and is designated as "appearance 1 of the cured product". Meanwhile, the case where the steel sheet is placed in a thermostatic tester in an atmosphere of 40°C and 95% RH is judged according to the following evaluation criteria and is designated as "appearance 2 of the cured product". Here, whitening in the evaluation criteria means that the periphery of the droplet of the cyanoacrylate composition turns white when the composition cures. The appearance of the cured product of the cyanoacrylate composition is preferably "good".

### Evaluation criteria

Good ··· No problem is found.
Acceptable ··· Whitening is observed inside the range in which the cyanoacrylate composition has been dropped.
Poor ··· Whitening is observed also at the outside of the range in which the cyanoacrylate composition has been dropped.

### [Appearance of adherend]

The test is performed in an atmosphere of 25°C and 55% RH. On a test piece that is 2.0 mm thick × 25 mm wide × 100 mm long and is made of ABS (acrylonitrile-butadiene-styrene copolymer), 15 mg of a curing accelerator was dropped, the test piece was left standing for 24 hours, and the state of the adherend in the region in which the curing accelerator had been dropped was observed according to the following evaluation criteria as "appearance of the adherend". The appearance of the adherend is preferably "good".

### Evaluation criteria

Good ··· No change is observed.
Acceptable ··· The adherend is slightly distorted and dissolved.
Poor ··· The adherend is eroded and cracked.

### [Setting time]

The test is performed in an atmosphere of 25°C and 55% RH. Test pieces made of four materials of NBR (acrylonitrile-butadiene copolymer), a steel sheet (SPCC-SD), ABS (acrylonitrile-butadiene-styrene copolymer), and PBT (polybutylene terephthalate), each 2.0 mm thick × 25 mm wide × 100 mm long, were used as adherends. Two adherends are used, and a curing accelerator is dropped on one of the adherends and spread with a cotton swab, and thereafter, ThreeBond 1786 manufactured by ThreeBond Co., Ltd. is dropped as a cyanoacrylate composition on the region to which the curing accelerator has been applied. The other adherend is laminated on the adherend to secure an adhesion area of 25 mm × 10 mm, and the adherends are held with a hand. At intervals of 1 second from the start to 10 seconds, and at intervals of 10 seconds on and after 11 seconds, one of the adherends is fixed by hand and the other adherend is lightly pulled in the shearing direction to confirm whether the adherends are fixed, and the time when the other adherend does not move any more is designated as "setting time (seconds)". For NBR, the steel sheet, and ABS, the setting time is preferably 5 seconds or less. Since PBT is a hardly adhesive material, the setting time is preferably 200 seconds or less.

**[Table 2]**

| Test item | | Example 1 | Example 2 | Example 3 | Example 4 | Example 5 | Example 6 | Comparative Example 1 | Comparative Example 2 |
|---|---|---|---|---|---|---|---|---|---|
| Appearance 1 of cured product | | good | good | good | good | good | good | good | good |
| Appearance 2 of cured product | | good | good | good | good | good | good | poor | poor |
| Appearance of adherend | | good | good | good | good | good | good | good | good |
| Setting time | NBR | 3 | 3 | 3 | 3 | 3 | 3 | 3 | 3 |
| | Steel sheet | 4 | 4 | 4 | 5 | 5 | 5 | 4 | 4 |
| | ABS | 2 | 2 | 2 | 2 | 2 | 2 | 2 | 2 |
| | PBT | 120 | 120 | 120 | 150 | 150 | 150 | 40 | 40 |

| Test item | | Comparative Example 3 | Comparative Example 4 | Comparative Example 5 | Comparative Example 6 | Comparative Example 7 | Comparative Example 8 | Comparative Example 9 | Reference Example 1 |
|---|---|---|---|---|---|---|---|---|---|
| Appearance 1 of cured product | | good | good | good | good | good | good | good | good |
| Appearance 2 of cured product | | poor | poor | poor | poor | poor | poor | acceptable | good |
| Appearance of adherend | | good | acceptable | acceptable | poor | good | poor | poor | good |
| Setting time | NBR | 3 | 4 | 4 | 7 | 3 | 3 | 3 | 6 |
| | Steel sheet | 4 | 4 | 8 | 12 | 10 | 6 | 8 | 10 |
| | ABS | 2 | 2 | 2 | 2 | 2 | 2 | 2 | 3 |
| | PBT | 60 | 90 | 110 | 50 | 120 | 180 | 180 | 540 |

In the comparison of Examples 1 to 6 with Comparative Examples 1 to 3 and 6 to 8, it was confirmed that whitening is observed in the appearance 2 of the cured product in the comparative examples containing the component (A') in addition to the component (A), although a clear reason is unknown. Furthermore, in the comparison of Examples 1 to 6 with Comparative Examples 4 and 5, it was confirmed that whitening is observed in the appearance 2 of the cured product in the comparative examples containing only the component (A') and not containing the component (A), although a clear reason is unknown. In contrast, in the examples containing the component (A) and not containing the component (A'), whitening was not observed even in the appearance 2 of the cured product. The appearance 1 of the cured product is "good" in all of Examples 1 to 6 and Comparative Examples 1 to 7, but the appearance 2 of the cured product also needs to be "good" in order that the cyanoacrylate composition can be used even in a high-temperature and high-humidity environment.

In addition, in Comparative Examples 4 to 6, 8, and 9, it was confirmed that when the rate of methanol and/or 4-hydroxy-4-methyl-2-pentanone is relatively high with respect to the entire composition, "acceptable" or "poor" appears in the appearance of the adherend, so that the adherend is greatly damaged.

As a curing accelerator for an object to be cured, such as a cyanoacrylate composition, a combination of an amine compound and an organic solvent is mainly used. However, as in the present invention, it has become possible to realize a curing accelerator that does not contain an amine compound, in particular, by combining alkaline electrolyzed water having a pH higher than 7 with a specific organic solvent. Therefore, the technique according to the present invention may serve as one of measures against VOCs and environmental measures that have been taken in various industries and fields in recent years. Furthermore, since the curing accelerator according to the present invention can reduce damage to an adherend, it can be used for various adherends.

## Claims

1. Use of a composition that contains a component (A) and a component (B) below and does not contain a solvent having a boiling point higher than 120°C in accelerating curing of a monomer:
the component (A): a solvent having 2 or more carbon atoms in one molecule and having a boiling point of 25 to 120°C; and
the component (B): water.

2. The use according to claim 1, wherein the composition consists only of the component (A) and the component (B).

3. The use according to claim 1 or 2, wherein the component (A) contains one or more selected from the group consisting of an alcohol, a ketone compound, and an ester compound.

4. The use according to claim 3, wherein the component (A) contains ethanol, n-propanol, n-butanol, or acetone.

5. The use according to any one of claims 1 to 3, wherein a mass ratio of the component (A) to the component (B) in the composition is 10 : 90 to 50 : 50.

6. The use according to any one of claims 1 to 5, wherein a pH of the component (B) is higher than 7.

7. The use according to any one of claims 1 to 6, wherein the monomer includes a cyanoacrylate compound.

8. The use according to any one of claims 1 to 7, wherein the curing is performed by applying the monomer and the composition are applied to the human body.

9. The use according to claim 8, wherein the curing is performed by applying the monomer and the composition are applied to the human eyelashes.

10. An instant adhesive kit comprising:
a composition that contains a component (A) and a component (B) below and does not contain a solvent having a boiling point higher than 120°C; and
a composition containing a monomer:
the component (A): a solvent having 2 or more carbon atoms in one molecule and having a boiling point of 25 to 120°C; and
the component (B): water.

11. The instant adhesive kit according to claim 10, wherein the composition consists only of the component (A) and the component (B).

12. The instant adhesive kit according to claim 10 or 11, wherein the component (A) contains one or more selected from the group consisting of an alcohol, a ketone compound, and an ester compound.

13. The instant adhesive kit according to any one of claims 10 to 12, wherein a mass ratio of the component (A) to the component (B) in the composition is 10 : 90 to 50 : 50.

14. The instant adhesive kit according to any one of claims 10 to 13, wherein a pH of the component (B) is higher than 7.

15. The instant adhesive kit according to any one of claims 10 to 14, wherein the monomer includes a cyanoacrylate compound.
